# EUROPEAN PATENT SPECIFICATION

(11) **EP 3 773 306 B1**
(45) Date of publication and mention of the grant of the patent: **04.12.2024**
(21) Application number: 19714724.2
(22) Date of filing: 19.03.2019
(51) Int. Cl.: A61B 34/30, A61B 46/10

(54) **SURGICAL DRAPE**
CHIRURGISCHES TUCH
CHAMP OPÉRATOIRE

(30) Priority: 26.03.2018 GB 201804805
(43) Date of publication of application: 17.02.2021
(73) Proprietor: CMR Surgical Limited, Cambridge CB24 9NG (GB)
(72) Inventor: MCBRIEN, Dominic Martin, Cambridge, Cambridgeshire CB24 9NG (GB); HIRD, Aidan, Cambridge, Cambridgeshire CB24 9NG (GB); MARSHALL, Keith, Cambridge, Cambridgeshire CB24 9NG (GB)
(74) Representative: Slingsby Partners LLP
(86) International application number: PCT/GB2019/050761
(87) International publication number: WO 2019/186112

(56) References cited:
- EP-A2- 2 151 211
- WO-A1-2017/015207
- KR-A- 20110 036 452
- US-A- 5 515 868
- US-A1- 2015 374 442
- US-A1- 2017 290 632

## Description

The present disclosure relates to surgical drapes, in particular to surgical robot drapes, for example surgical robot drapes that can allow access to a robot draped by the drape.

### BACKGROUND

It is known to use robots for assisting and performing surgery. A surgical robot typically comprises a base on which a robot arm can be supported. An instrument couples to the end of the robot arm distal of the base and supports an end effector for engaging in a medical procedure. The robot arm comprises multiple flexible joints along its length, which are used to locate the surgical instrument in a desired location relative to a patient. The surgical instrument can penetrate the body of the patient at a port so as to access a surgical site. The end effector can be used to perform the medical procedure at the surgical site.

The surgical instrument needs to be sterile. The robot need not be sterile. Typically, a surgical drape is provided for covering the robot. The surgical drape provides a sterile barrier between the surgical instrument and the robot. The drape provides a boundary between the robot and the sterile field in which the robot is positioned, for example an operating room.

The drape can be provided in the form of a large loose sheet of an impermeable membrane. Covering the robot arm with such a large loose sheet can mean that the sheet is loose about the robot arm and can interfere with the procedure being carried out.

When the drape covers the robot, it is desirable that the outer surface of the drape (i.e. the side of the drape facing the operating room) is sterile. In an operating room, a non-sterile person may require access to the robot to position the robot and/or adjust controls on the robot.

WO 2017/015207 describes robotic system drapes, including for surgical systems. The drape can comprise an opening in a preselected position to provide access to an arm or base. The opening can be closed. US 2017/290632 describes a surgical drape which has a slit at the back which can be closed to retain the drape on the robot. KR 2011 0036452 describes a surgical drape which comprises a magnet for holding the drape in position. US 5515868 describes a non-robotic surgical drape. The drape can be opened along a slit extending from an aperture to the edge of the drape. EP 2151211 describes a non-robotic drape that can be opened along a cut line. The cut line is formed by severing the sheet along the line and then securing the edges of the cut together with a strip. US 2015/374442 describes a non-robotic drape that is perforated so that it can be separated into parts.

### SUMMARY

The invention is as defined in claim 1, with further embodiments disclosed in the dependent claims. According to an aspect of the disclosure there is provided a surgical robot drape for providing a sterile barrier over a portion of a surgical robot, the drape comprising a plurality of accessibility portions spaced apart from one another in the drape for providing selective access through the drape at the respective locations of the accessibility portions, each accessibility portion comprising a separation line joining neighbouring portions of the drape and being configured to permit the separation of the neighbouring portions of the drape about the separation line for allowing access through an opening thereby formed in the drape.

The drape may comprise the same number of accessibility portions as a number of orientations at which a surgical robot can be draped by the drape. The accessibility portions may be equally spaced about the drape, in a direction about which the drape can be selectively oriented to drape the surgical robot.

Each accessibility portion may be configured to preferentially tear along the separation line under action of a force. The separation line may comprise a weakened portion of material relative to the neighbouring portions of the drape. The separation line may comprise material thinner than that of the neighbouring portions.

The separation line may comprise perforations. The perforations may extend through the material.

The separation line may comprise a removable tear strip of relatively stronger material than the neighbouring portions.

The separation line may enclose a removable section of the drape. The separation line may extend to an edge of the drape.

The accessibility portion may be formed unitarily with the drape.

The accessibility portion may comprise a portion of material overlapping with the separation line. The overlapping material may be formed by a fold in the material.

The separation line may comprise an adhesive join between the neighbouring portions. The adhesive join may comprise a portion of separation material adhesively joined to each of the neighbouring portions.

The accessibility portion may comprise a tab for holding by a user. The fold in the material may comprise the tab.

The drape may comprise a flexible sheet having a skirt portion configured to, in a first configuration, drape the portion of the surgical robot to provide a sterile barrier thereover; and a retention arrangement configured for retaining the skirt portion in a second configuration in which the skirt portion exposes the portion of the surgical robot for providing access thereto.

The drape may comprise a weight for urging the skirt portion towards the first configuration. The retention arrangement may be provided at or towards a lower edge of the drape. The retention arrangement may be configured for retaining the skirt portion at least one of mechanically and magnetically.

The retention arrangement may be configured for releasable engagement with a further retention arrangement for retaining the skirt portion in the second configuration.

The drape may comprise a fold or tab, whereby a user can hold the drape via the fold or tab without thereby affecting the sterility of the remainder of the drape. The retention arrangement may comprise the weight and/or the fold or tab.

The drape may comprise a plurality of retention arrangements. The drape may comprise the same number of retention arrangements as a number of orientations at which a surgical robot can be draped by the drape. The retention arrangements may be equally spaced about the drape, in a direction about which the drape can be selectively oriented to drape the surgical robot.

At least one of the neighbouring portions of each accessibility portion may comprise a respective retention arrangement. At least one of the neighbouring portions of each accessibility portion may comprise a respective weight.

According to another aspect, there is provided a surgical robot drape for providing a sterile barrier over a portion of a surgical robot, the drape comprising a flexible sheet having a skirt portion configured to, in a first configuration, drape the portion of the surgical robot to provide a sterile barrier thereover; and a retention arrangement configured for retaining the skirt portion in a second configuration in which the skirt portion exposes the portion of the surgical robot for providing access thereto.

The drape may be configured to permit the skirt portion to be moved between the first configuration and the second configuration without thereby affecting the sterility of the drape.

The drape may comprise a weight for urging the skirt portion towards the first configuration. The weight may be fast with the skirt portion. The weight may be provided at or towards a lower edge of the drape. The weight may be provided within a fold or pocket of the drape. The weight may comprise a weighted strip, the weighted strip being provided along at least a portion of the lower edge of the drape.

The retention arrangement may be provided at or towards a lower edge of the drape. The retention arrangement may be at least one of welded and bonded to material of the drape. The retention arrangement may be at least one of welded and bonded to the skirt portion.

The retention arrangement may be configured for retaining the skirt portion through material of the drape.

The retention arrangement may be configured for retaining the skirt portion at least one of mechanically and magnetically.

The retention arrangement may be configured for releasable engagement with a further retention arrangement for retaining the skirt portion in the second configuration. The drape may comprise a first retention feature forming at least part of the further retention arrangement. The first retention feature may be at least one of welded and bonded to material of the drape and/or held fast relative to material of the drape in a fold or pocket of the drape.

A second retention feature may be provided on the surgical robot to be draped by the drape.

The retention arrangement may comprise a first magnetic material. The further retention arrangement may comprise a second magnetic material for magnetic attachment to the first magnetic material.

The retention arrangement may comprise one part of a hook and loop fastener and the further retention arrangement may comprise a cooperating part of the hook and loop fastener.

The retention arrangement may comprise a clip.

The drape may comprise a fold or tab, whereby a user can hold the drape via the fold or tab without thereby affecting the sterility of the remainder of the drape. The retention arrangement may comprise the fold or tab.

The retention arrangement may comprise the weight.

The drape may comprise a plurality of retention arrangements. The drape may comprise the same number of retention arrangements as a number of orientations at which a surgical robot can be draped by the drape. The retention arrangements may be equally spaced about the drape, in a direction about which the drape can be selectively oriented to drape the surgical robot.

According to another aspect, there is provided a surgical robotic system comprising a surgical robot and a surgical robot drape as described herein, in which the drape is disposed about the surgical robot for providing a sterile barrier over at least a portion thereof.

This Summary is provided to introduce a selection of concepts in a simplified form that are further described below in the Detailed Description. The mention of features in this Summary does not indicate that they are key features or essential features of the invention or of the claimed subject matter, nor is it to be taken as limiting the scope of the claimed subject matter. The scope of the invention is defined by the appended claims.

### BRIEF DESCRIPTION OF THE DRAWINGS

The present invention will now be described by way of example with reference to the accompanying drawings. In the drawings:
figure 1 illustrates an example of a surgical robot;
figure 2 illustrates another example of a surgical robot;
figure 3 illustrates an example of a surgical robot covered by a drape;
figure 4a illustrates another example of a surgical robot covered by a drape;
figure 4b illustrates the example of figure 4a in a different orientation;
figure 5 illustrates another example of a surgical robot covered by a drape;
figure 6 illustrates the example of figure 5 with the drape in a different configuration;
figure 7 illustrates another example of a surgical robot covered by a drape;
figure 8 illustrates the example of figure 7 with the drape in a different configuration;
figure 9 illustrates an example of a drape comprising a separation line;
figure 10 illustrates another example of a drape comprising a separation line;
figure 11 illustrates another example of a drape comprising a separation line;
figures 12a, 12b and 12c illustrate examples of an adhesive join;
figure 13 illustrates another example of a drape comprising a separation line;
figure 14 illustrates another example of a surgical robot covered by a drape;
figure 15 illustrates the example of figure 14 with the drape in a different configuration;
figure 16 illustrates an example of a portion of a surgical robot covered by a drape;
figures 17a and 17b illustrate configurations of a drape comprising a retention arrangement;
figures 18a, 18b and 18c illustrate other configurations of a drape comprising a retention arrangement;
figures 19a and 19b illustrate other configurations of a drape comprising a retention arrangement;
figure 20a illustrates another example of a drape comprising a retention arrangement;
figure 20b illustrates the example of figure 20a in a different configuration; and
figure 21 illustrates an example of a drape comprising accessibility portions and retention arrangements.

### DETAILED DESCRIPTION

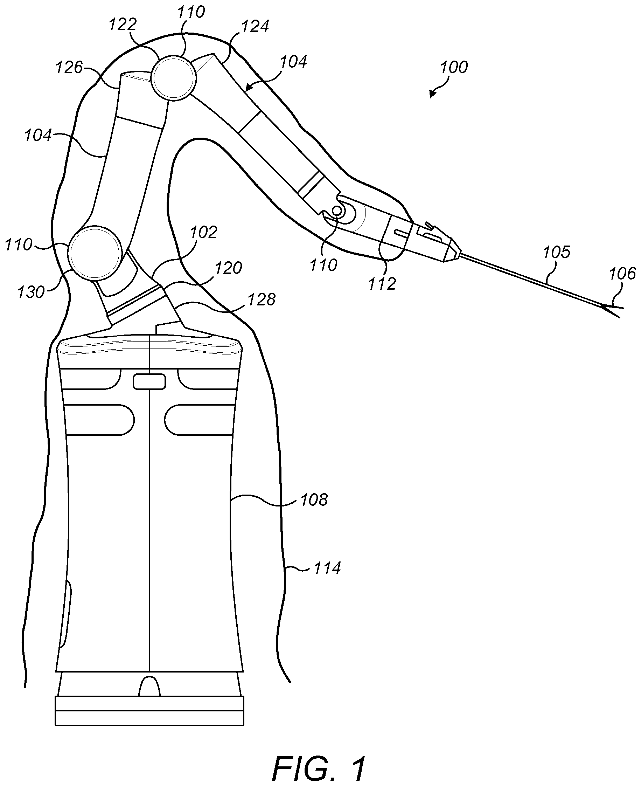

Figure 1 illustrates a surgical robot 100 which comprises a robot arm. The robot arm comprises a base 102 of the arm and a plurality of arm segments 104. The surgical robot comprises an instrument 105 coupled to an end of the robot arm, the instrument comprising an end effector 106. The base supports the remainder of the robot arm on, for example, the operating theatre floor, the operating theatre ceiling or a trolley or cart 108. The arm extends from the base of the arm to a distal end of the arm. The instrument is coupled to the distal end of the arm. The arm is articulated by means of multiple flexible joints 110 along its length, which are used to locate the surgical instrument in a desired location relative to a patient. The joints 110 provide articulation between parts of the surgical robot attached to either side of each joint, for example two arm segments 104 or the base 102 of the arm and a mount (such as a cart or ceiling mount) to which the robot arm is mounted.

The surgical instrument 105 is attached to the distal end 112 of the robot arm. The surgical instrument can penetrate the body of the patient at a port so as to access a surgical site. At its distal end, the instrument comprises the end effector 106 for engaging in a medical procedure.

The surgical robot 100 is shrouded by a surgical drape 114 to provide a sterile boundary between the surgical instrument (which must be sterile) and the robotic arm and cart 108 (which may not be sterile). The drape provides a boundary between the robot and the sterile field in which the robot is positioned (for example an operating theatre).

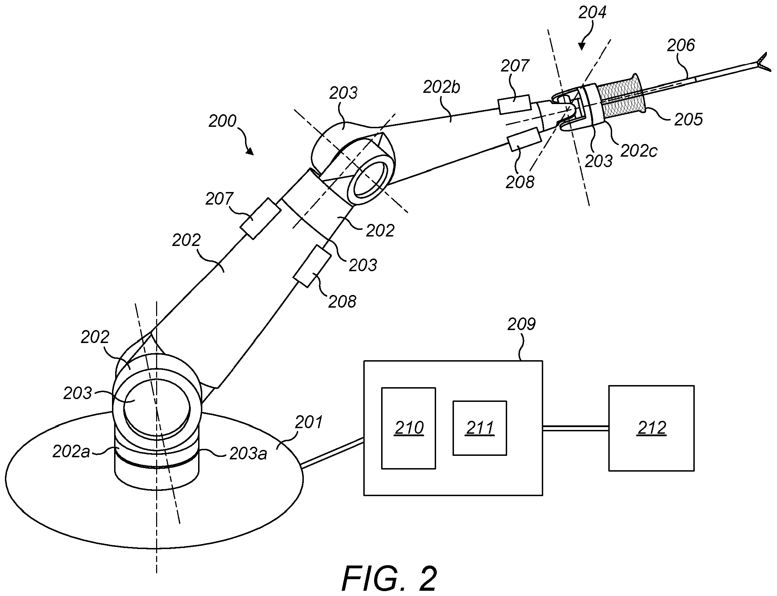

Figure 2 illustrates a surgical robot having an arm 200 which extends from a mount 201. The mount 201 can be part of a trolley or cart, for example a mount at the top of such a cart. The arm comprises a number of rigid limbs 202. The limbs are coupled by revolute joints 203. The most proximal limb 202a is coupled to the mount by a proximal joint 203a. It and the other limbs are coupled in series by further ones of the joints 203. Suitably, a wrist 204 is made up of four individual revolute joints. The wrist 204 couples one limb (202b) to the most distal limb (202c) of the arm. The most distal limb 202c carries an attachment 205 for a surgical instrument 206. Each joint 203 of the arm has one or more motors 207 which can be operated to cause rotational motion at the respective joint, and one or more position and/or torque sensors 208 which provide information regarding the current configuration and/or load at that joint. Suitably, the motors are arranged proximally of the joints whose motion they drive, so as to improve weight distribution. For clarity, only some of the motors and sensors are shown in figure 2. The arm may be generally as described in our published patent application WO2015132549.

The arm terminates in the attachment 205 for interfacing with the instrument 206. An instrument interface can be mounted to a drive assembly interface of the attachment. The instrument may have a diameter less than 8mm. Suitably, the instrument has a 5mm diameter. The instrument may have a diameter which is less than 5mm. The instrument diameter may be the diameter of the shaft. The instrument diameter may be the diameter of the profile of the articulation. Suitably, the diameter of the profile of the articulation matches or is narrower than the diameter of the shaft. The attachment 205 comprises a drive assembly for driving articulation of the instrument. Movable interface elements of the drive assembly interface mechanically engage corresponding movable interface elements of the instrument interface in order to transfer drive from the robot arm to the instrument. One instrument is exchanged for another several times during a typical operation. Thus, the instrument is attachable to and detachable from the robot arm during the operation.

The instrument 206 comprises an end effector for performing an operation. The end effector may take any suitable form. For example, the end effector may be smooth jaws, serrated jaws, a gripper, a pair of shears, a needle for suturing, a camera, a laser, a knife, a stapler, a cauteriser, a suctioner and so on. The instrument comprises an articulation between the instrument shaft and the end effector. The articulation comprises several joints which permit the end effector to move relative to the shaft of the instrument. The joints in the articulation are actuated by driving elements, such as cables. These driving elements are secured at the other end of the instrument shaft to the interface elements of the instrument interface. Thus, the robot arm transfers drive to the end effector as follows: movement of a drive assembly interface element moves an instrument interface element which moves a driving element which moves a joint of the articulation which moves the end effector.

Controllers for the motors, torque sensors and encoders are distributed within the robot arm. The controllers are connected via a communication bus to a control unit 209. The control unit 209 comprises a processor 210 and a memory 211. The memory 211 stores in a non-transient way software that is executable by the processor to control the operation of the motors 207 to cause the arm 200 to operate in the manner described herein. In particular, the software can control the processor 210 to cause the motors (for example via distributed controllers) to drive in dependence on inputs from the sensors 208 and from a surgeon command interface 212. The control unit 209 is coupled to the motors 207 for driving them in accordance with outputs generated by execution of the software. The control unit 209 is coupled to the sensors 208 for receiving sensed input from the sensors, and to the command interface 212 for receiving input from it. The respective couplings may, for example, each be electrical or optical cables, or may be provided by a wireless connection. The command interface 212 comprises one or more input devices whereby a user can request motion of the end effector in a desired way. The input devices could, for example, be manually operable mechanical input devices such as control handles or joysticks, or contactless input devices such as optical gesture sensors. The software stored in the memory 211 is configured to respond to those inputs and cause the joints of the arm and instrument to move accordingly, in compliance with a pre-determined control strategy. The control strategy may include safety features which moderate the motion of the arm and instrument in response to command inputs. Thus, in summary, a surgeon at the command interface 212 can control the instrument 206 to move in such a way as to perform a desired surgical procedure. The control unit 209 and/or the command interface 212 may be remote from the arm 200.

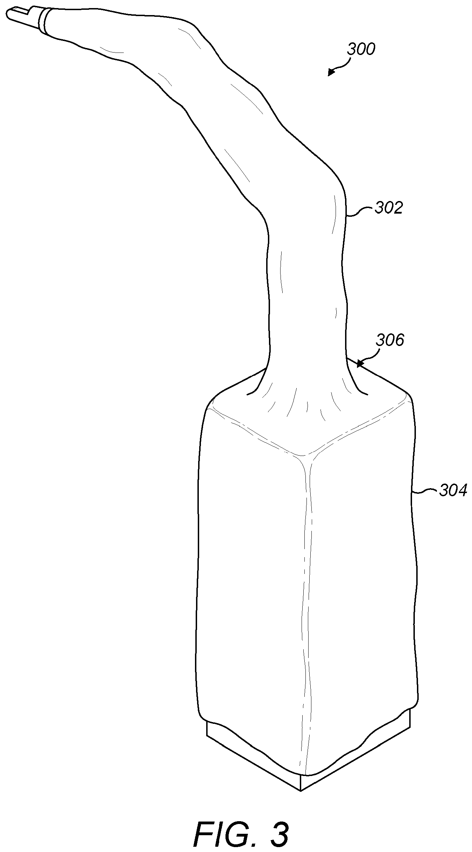

During an operation or surgical procedure, the surgical robot is shrouded in a sterile drape (an example of which is illustrated at 300 in figure 3) to provide a sterile barrier between the non-sterile surgical robot and the sterile operating environment. The portion of the robot that is covered by the drape then need not be sterile. The surgical instrument is sterilised before being attached to the surgical robot. The sterile drape is typically constructed of a plastic sheet, for example made of polyester, polypropylene, polyethylene or polytetrafluoroethylene (PTFE). Suitably, the drape is flexible and/or deformable. This can assist in the drape shrouding the robot arm without interfering in the location and/or movement of the robot arm and/or instruments during the surgical procedure.

In the example illustrated in figure 3, the drape comprises a part for draping an arm 302 and a part for draping a cart 304. The arm part of the drape and the cart part of the drape may be unitary with one another, as illustrated. An arm drape and a cart drape may be separately provided. To reduce the chances of the drape interfering in the location and/or movement of the robot arm and/or instruments during the surgical procedure, the drape can be of a generally close-fitting form to one or both of the robot arm and the cart. The robot arm and cart will be of known dimensions, so the drape can be configured, or manufactured, so as to closely fit about the robot arm or cart with which it is desired to use the drape.

As can be seen from the illustration in figure 3, the cart can be shaped substantially as a rectangular prism. The cart part of the drape suitably also adopts a similar shape so as to closely cover the cart. This means that the cart part of the drape has particular orientations at which it can be applied over the cart. As illustrated, for a rectangular prism-shaped cart, there will be four orientations, separated by 90 degrees, at which the cart part of the drape can be applied over the cart.

Once applied over the cart, the cart part of the drape will not be able to substantially twist, or rotate, with respect to the cart. The cart part of the drape will at least substantially maintain its orientation with respect to the cart. This can mean that, for a unitary drape as illustrated in figure 3, the drape needs to be correctly oriented when applying the drape over the robot for it to correctly fit the cart. Surgical drapes are typically applied to surgical robots (such as the one illustrated in figure 1) by first applying a part of the drape to the distal part of the arm, then unfolding or unfurling the remainder of the drape along the arm and over the cart. Thus the member of operating room (OR) staff applying the drape needs to correctly orient the cart part of the drape before applying the arm part of the drape to the robot arm. This is difficult and takes time. If the drape is not initially oriented correctly, it must be re-oriented partway through draping the robot. This can be awkward. As the drape unfurls, the side that comes into contact with the robot is then no longer sterile. This means that the drape cannot be rolled back up again for re-applying to the robot. Thus, in some situations the drape must be discarded and the process started again. This is wasteful, both of time and of materials.

The distal end of the arm can be controlled to be in different configurations relative to the remainder of the arm, for example by being rotated about a joint proximal of the distal end. Thus the distal end of the arm can be in different configurations relative to the cart to which the arm can be mounted. Therefore applying the drape to the distal end of the robot arm in the correct orientation (for draping the robot arm), and then unfurling the drape along the arm, can mean that the drape is not ideally orientated relative to the cart. Further, the joints of the arm may be articulated such that the arm can adopt a variety of configurations.

The drape may therefore need to be twisted to correctly fit over one or both of the arm and cart. This complicates the process of applying the drape to the robot, and can mean that undesirable forces such as shear forces are applied to the drape.

In some situations, applying forces to the drape, such as by twisting the drape, can adversely affect the integrity of the drape. For example, the drape may rip or tear, or otherwise rupture, thus potentially compromising the sterile barrier. Such a rupture might occur at a portion which experiences shear forces. A rupture in the drape would risk exposing the sterile field to the non-sterile robot. The sterile barrier may additionally or alternatively be compromised by the drape being thinned, or worn away, over time as well as by the drape rupturing. It is therefore important to maintain the integrity of the drape to ensure that the sterile barrier is not compromised.

A force within the drape material may be transferred to a part of the robot, for example the part of the robot adjacent the drape material. This can impair the performance of the robot. For example, the force on the robot caused by the drape can restrict free movement of the robot. The force on the robot caused by the drape can affect force feedback measurements which might sense forces on one or more portion of the robot. Thus the forces caused by the drape might introduce inaccuracies in such force feedback measurements.

The cart drape portion, or a separate cart drape, can be applied to the cart in one of four orientations in the example shown. This is because, in the illustrated example, the cart is of a generally rectangular prism shape. In other examples, the cart drape portion may have a different number of orientations with respect to the cart. The cart drape portion is rotationally fast with the cart in the sense that turning the cart about its vertical axis will cause the cart drape portion to also turn about this axis. The cart drape portion will turn together with the cart. The cart drape portion will not substantially rotate relative to the cart itself. Similarly, where the cart is retained in a fixed orientation, the cart drape portion will also be substantially retained in that orientation. The cart drape portion need not be attached to the cart. The shape of the cart drape portion may restrict relative movement between the cart drape portion and the cart. I.e. the cart drape portion can be configured to restrict relative movement between the cart drape portion and the cart.

There are requirements about which member of the operating room (OR) team can perform the draping of the robot, and how they do so, in order to maintain the sterile barrier over the robot. The OR team comprises sterile members of the team, such as a scrubbed medical practitioner. The sterile members of the team may only touch the sterile (patient-facing) surface of the drape. This is the outer surface of the drape once applied to the robot. However, the sterile members of the team may not touch the robot (which is not sterile) during the draping procedure, or indeed during a surgical procedure once the robot has been draped.

In contrast, non-sterile members of OR staff, such as circulating members of staff, may not touch the sterile surface of the drape - if they do so, then the drape becomes (at least partially) non-sterile and may need to be replaced for a surgical procedure to commence. The non-sterile members of staff may touch the robot (which is not sterile). However, once the robot has been draped by the drape, there is a difficulty in providing access to the robot, or a portion thereof, to the non-sterile member of staff, without thereby affecting the sterility of the drape. For instance, the non-sterile member of staff cannot simply hold and pull up the drape, or cut a hole in the drape, since by contacting the drape in this way they would render the portions that they touch non-sterile. It is possible for a non-sterile member of staff to use a tool to move the drape and/or to cut a hole in the drape, where the tool has a sterile end for contacting the drape. However, this adds complexity to the operation, and means that additional items (the tools) need to be sterilised and provided in the OR for use by the non-sterile staff. Also, where a tool is used, the tool may contact the (non-sterile) robot rendering the tool non-sterile. In such a case the tool may only be used once before needing replacing or sterilising. This is inconvenient.

The surgical robot, or a cart of a surgical robot, as illustrated, may be positioned adjacent an operating table in different orientations, depending on the procedure to be performed. Both sterile and non-sterile members of staff will preferably have access to move the robot, or cart, and adjust the controls on the cart. For example, once the robot has been draped, a sterile member of staff can hold the robot via the sterile outer surface of the drape to move the robot into the desired position. A non-sterile member of staff will require access to the robot without being able to touch the sterile surface of the drape.

Whichever side of the robot, or cart, is facing away from the operating table can be considered to be the non-sterile side, and a non-sterile member of staff can be permitted to access that side of the robot. One way of permitting such access is to provide a window or aperture in the drape. If the drape comprises a pre-cut window, this will lead to difficulty in applying the drape to the robot since it needs to be known in advance to which side of the robot access is needed, and so which side of the robot the window in the drape needs to align with. As discussed, this makes getting the drape (whether provided in a separate cart drape, or a unitary arm and cart drape) into the correct orientation for applying the drape difficult and confusing.

Additionally, if there is a pre-cut window in the drape, the drape may become more difficult to handle. For example, users would be able inadvertently to put their hand or arm through the window thereby breaching the sterile barrier. For example, a sterile member of staff applying the drape might inadvertently touch the robot through the window rendering themselves (partly) non-sterile, and necessitating additional action, such as re-scrubbing, which is time consuming and inconvenient.

It is therefore desirable to provide a drape that can be applied to a surgical robot, or to at least a portion of a surgical robot, with a reduced need to rearrange and/or orient the robot (such as the arms of the robot or the cart of the robot) or the drape prior to applying the drape.

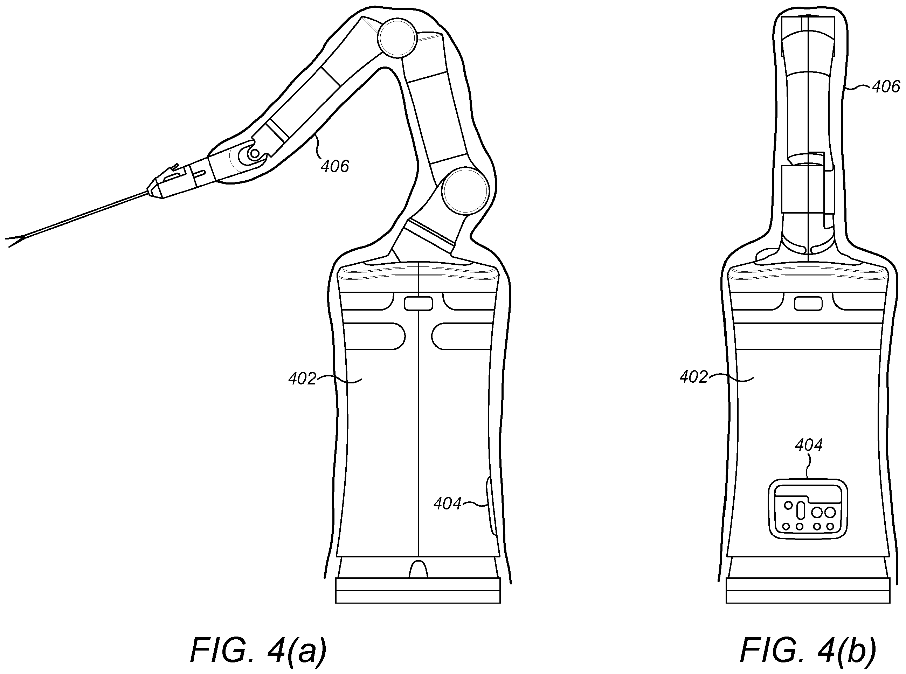

In particular, with reference to figures 4a and 4b, the cart 402 may comprise connectors for coupling to a control system. The connectors may be located within a connector panel 404. As illustrated, the connector panel 404 is provided in one of the four faces of the cart 402. The connector panel 404 should be accessible following draping of the cart by the drape 406. The connector panel 404 is preferably accessible to a non-sterile member of staff, for example to permit cables to be connected to and/or disconnected from the connector panel.

As mentioned, the cart drape can be in the form of a relatively close-fitting drape. The cart drape may therefore not be rotatable relative to the cart once applied to the cart. In this example, where the cart comprises four faces, the drape may be applied in one of four orientations relative to the cart. In other examples, the cart may comprise more or fewer faces, and the drape may be correspondingly shaped to fit to such a cart. To avoid needing to know in advance which part of the drape will be adjacent each of the four faces of the cart (or such other number as appropriate for the cart/robot), the drape may be provided with accessibility portions (e.g. arrangements permitting access to the robot) for providing access through the drape. For example, an accessibility portion can provide access through the drape at its location. It is desirable for the accessibility portion to provide selective access. For instance it may be possible to select, such as by a user selecting, whether or not access is needed through an accessibility portion, and access only being provided where it is desired. In this way, the drape can avoid the drawbacks of having pre-cut windows in the drape. A plurality of accessibility portions may be provided in the drape. The accessibility portions may be spaced apart from one another in the drape. This configuration can reduce the need to orient the drape before applying the drape.

Each accessibility portion comprises a separation line joining neighbouring portions of the drape. The neighbouring portions may be separated from one another about the separation line. Each accessibility portion is configured to permit the separation of the neighbouring portions of the drape about the separation line for allowing access through an opening thereby formed in the drape. The separation of the neighbouring portions about the separation line suitably permits a separation of the neighbouring portions to either side of the line. The separation of the neighbouring portions may be lateral of the separation line.

Preferably, the number of accessibility portions in the drape corresponds to the number of orientations at which the robot can be draped by the drape. Thus, at whichever orientation the drape is applied, one of the accessibility portions will be located so as to provide access to the desired portion of the robot (such as a face of the cart).

Hence, in the example of a cart with four faces, as illustrated in figures 4a and 4b, the drape may be provided with four such accessibility portions. Since the cart as illustrated is generally square in horizontal cross-section, the drape may be provided with the four accessibility portions being equally spaced about the drape. The accessibility portions may be spaced from one another in the direction in which the drape can be selectively oriented to drape the surgical robot. In this way, at each orientation at which the drape can drape the surgical robot, one of the accessibility portions can provide access to a desired portion of the surgical robot.

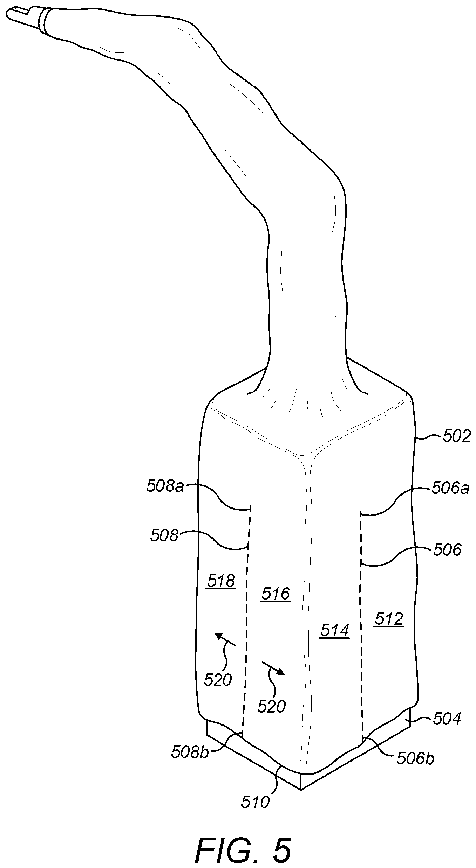

An example of a drape comprising accessibility portions is shown in figure 5. Figure 5 illustrates a drape 502 covering a robot 504. Two accessibility portions are visible in figure 5. It will be understood that two other accessibility portions are provided in the drape as illustrated, but which are not visible in the illustrated view. The accessibility portions comprise respective separation lines 506 508. As illustrated the separation lines are straight lines, though this need not be the case. For example, the separation lines may be curved lines, or some combination of curved and straight lines. The separation lines of different accessibility portions need not be the same shape, although it is convenient for them to be the same shape. As illustrated, the separation lines extend from respective upper ends 506a 508a to respective lower ends 506b 508b. The lower ends of the separation lines terminate at the lower edge of the drape 510. In some examples the separation lines may extend to other edges of the drape. In some examples the separation lines 506 508 need not extend to an edge of the drape.

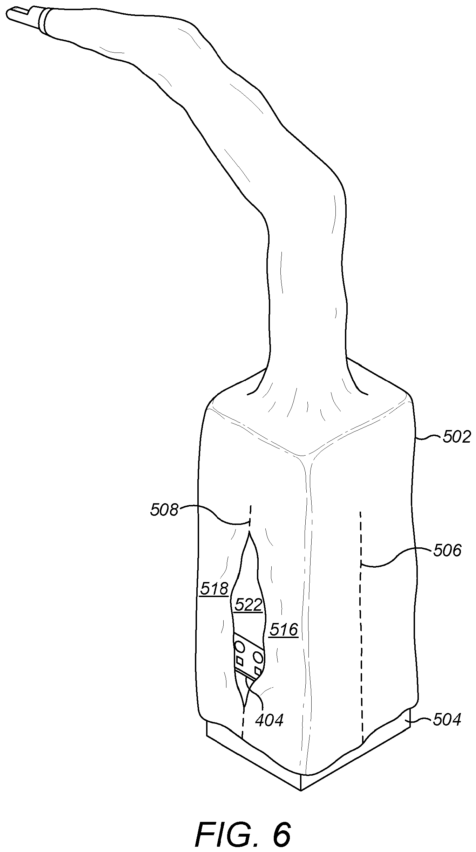

The separation line 506 of one of the accessibility portions permits the separation of a first pair of neighbouring portions 512 514 of the drape 502. The separation line 508 of another of the accessibility portions permits the separation of a second pair of neighbouring portions 516 518 of the drape 502. The separation line 508 can permit the second pair of neighbouring portions 516 518 to separate from one another in the directions of arrows 520. This is illustrated in figure 6.

Figure 6 shows a robot draped by a drape in the same orientation as that shown in figure 5. As illustrated in figure 6, the second pair of neighbouring portions 516 518 of the drape 502 have been separated from one another about the separation line 508 of one of the accessibility portions. An opening 522 has thereby been formed in the drape 502. The opening 522 provides access through the drape 502 to the robot. In the illustrated example, the opening 522 provides access to the connector panel 404.

The opening 522 does not extend to the lower edge 510 of the drape in the illustrated example. Thus, in this example, the lower edge 510 of the drape remains continuous, and the opening 522 takes the form of a window or aperture in the drape 502. In other examples, the neighbouring portions 516 518 can be separated along the entire length of the separation line 508, or along a portion of the length of the separation line from the lower end 508b. Where the separation line 508 extends to the edge of the drape, the separation of the neighbouring portions about the separation line can lead to the lower edge of the drape becoming discontinuous. For instance, in some examples, the lower portions of the neighbouring portions will no longer be joined together. This can result in each of the neighbouring portions comprising a flap. The flap may be folded to one side. This folding of the flap may provide a greater level of access through, or past, the drape. For example, by providing an opening 522 of a greater width than may otherwise be the case.

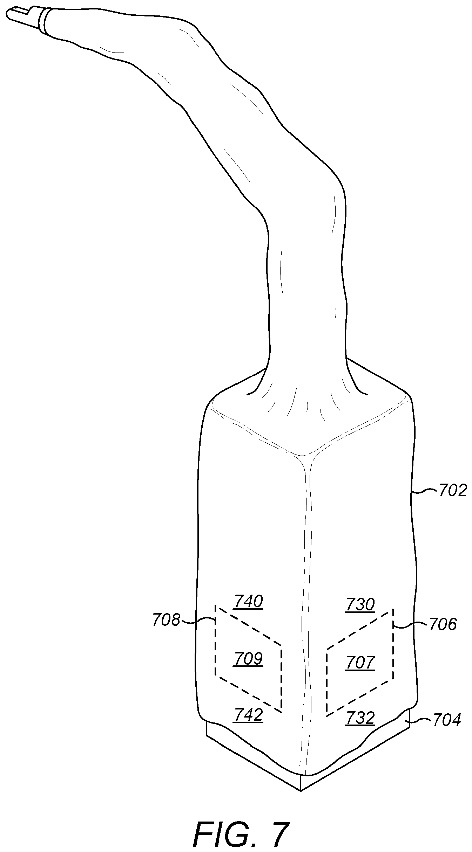

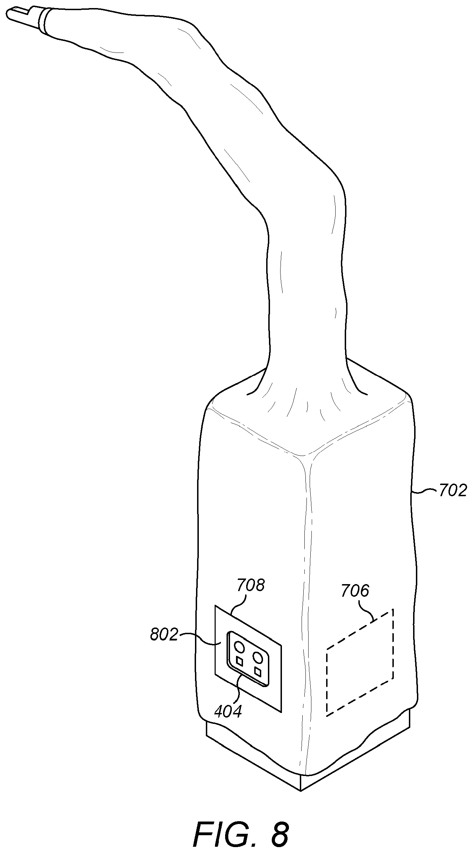

Referring now to figures 7 and 8, an alternative configuration of the accessibility portions will be described. Figure 7 illustrates a drape 702 covering a surgical robot 704. As in figure 5, two accessibility portions are visible in figure 7. One accessibility portion comprises separation line 706. Another accessibility portion comprises separation line 708. In this configuration of the accessibility portions, the separation lines 706 708 enclose respective removable sections of the drape 702. Separation line 706 encloses removable section 707. Separation line 708 encloses removable section 709. Similarly, other separation lines can enclose other removable sections.

The separation lines of the accessibility portions illustrated in figure 7 permit separation of neighbouring portions of the drape. In this example, removable section 707 is separable from portions 730 732 of the drape. Similarly, removable section 709 is separable from portions 740 742 of the drape. Separation of the neighbouring portions of the drape in this way results in the removable sections 707 709 being completely separable from the remainder of the drape 702. The removal of one of the removable sections forms a window or aperture in the drape. This is illustrated in figure 8.

Figure 8 shows the drape 702 of figure 7, with one of the removable sections 709 of the respective accessibility portion having been removed from the remainder of the drape. The removal of the removable section 709 forms an opening 802 in the drape. The opening 802 allows access through the drape to the robot 704. As illustrated, the opening 802 allows access to the connector panel 404.

The accessibility portions may be configured such that the accessibility portion will preferentially tear along the respective separation line. For example, where a force is applied to the accessibility portion, the separation line will tear more easily than the remainder of the material of the accessibility portion. This permits the opening to be formed in a known location, by permitting the neighbouring portions to separate about the separation line.

The separation lines of each accessibility portion may comprise a weakened portion of material. The weakened portion of material is suitably weakened compared to the neighbouring portions of the drape. For instance, the separation line may comprise material that is weaker than material of the neighbouring portions of the drape. This can assist in the preferential tearing of material along the separation line.

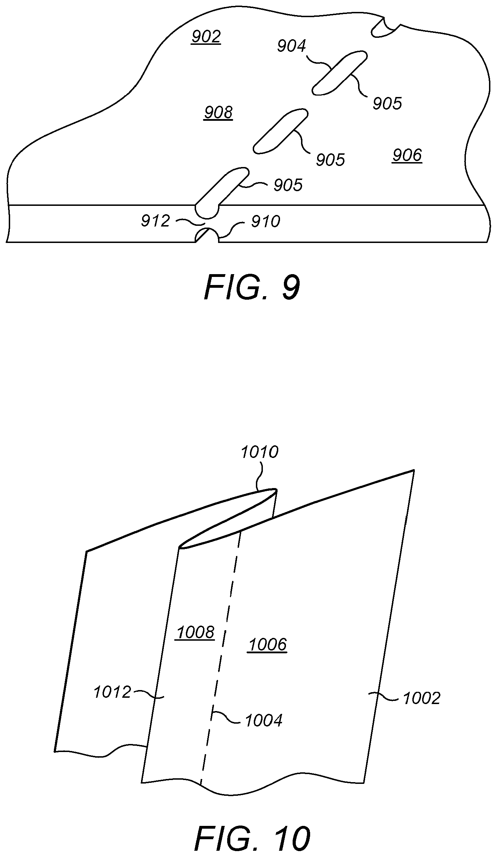

The material of the separation line may be the same material as that of the neighbouring portions, or it may be a different material. Where the separation line comprises a different material from the neighbouring portions, the material of the separation line may be a weaker material than that of the neighbouring portions. Where the separation line comprises the same material as the neighbouring portions, or different material to that of the neighbouring portions, the material of the separation line may be configured to be weaker than the material of the neighbouring portions. For example, the separation line may comprise thinner material than that of the neighbouring portions. The material of the separation line may comprise perforations. Reference is made to figure 9, showing a partial cut away view of a portion of an accessibility portion in a drape 902. A separation line 904 is provided between neighbouring portions 906 908 of material of the drape. The separation line 904 comprises perforations 905. The perforations comprise recesses 910 in either side of the material. In other examples, the recesses need only be provided to one side of the material. The perforations of figure 9 do not extend through the material. Instead, the recesses 910 are separated by a thinner strip of material 912, relative to the material of the neighbouring portions 906 908. In other examples, the perforations of the separation line may extend through the material.

It is desirable to maintain the sterile barrier at all of the accessibility portions until the neighbouring portions of one of the accessibility portions are separated along its separation line. This is because not all of the accessibility portions are likely to be used to provide access therethrough. The accessibility portions that are not used to provide access therethrough should be able to maintain the sterile barrier. For example, in the case where four accessibility portions are provided, all four should initially provide a sterile barrier over the robot when the drape is applied to the robot. When one of the accessibility portions is opened to provide access through the opening to the robot, this accessibility portion need no longer maintain the sterile barrier. However, the remaining, unopened, accessibility portions should still be able to maintain the sterile barrier.

Where there exists a fluid flow path through the separation line (for example where perforations extend through the material), it is useful to provide additional material to help maintain the sterile barrier. The additional material may also be provided in examples where a fluid flow path does not exist through the drape. The additional material may comprise material that overlaps with the separation line. The overlapping material may be material of the drape.

An example of the provision of overlapping material is illustrated in figure 10. Figure 10 shows an accessibility portion in a portion of a drape 1002. The accessibility portion comprises a separation line 1004, which permits separation of neighbouring portions 1006 1008 about the separation line 1004. In the illustrated example the separation line 1004 comprises perforations. In other examples the separation line 1004 may be of any suitable other configuration, for example the configurations discussed herein.

Overlapping material 1010 is provided. The overlapping material is provided behind the separation line (as illustrated). In other examples the overlapping material may be provided in front of the separation line. Provided the overlapping material behind the separation line can make it easier to locate the separation line. The overlapping material is provided in the illustrated example by way of a fold or pleat in the material of the drape. This configuration permits the accessibility portion, comprising the overlapping material, to be easily formed. This configuration assists in forming the accessibility portion, comprising the overlapping material, in a unitary manner.

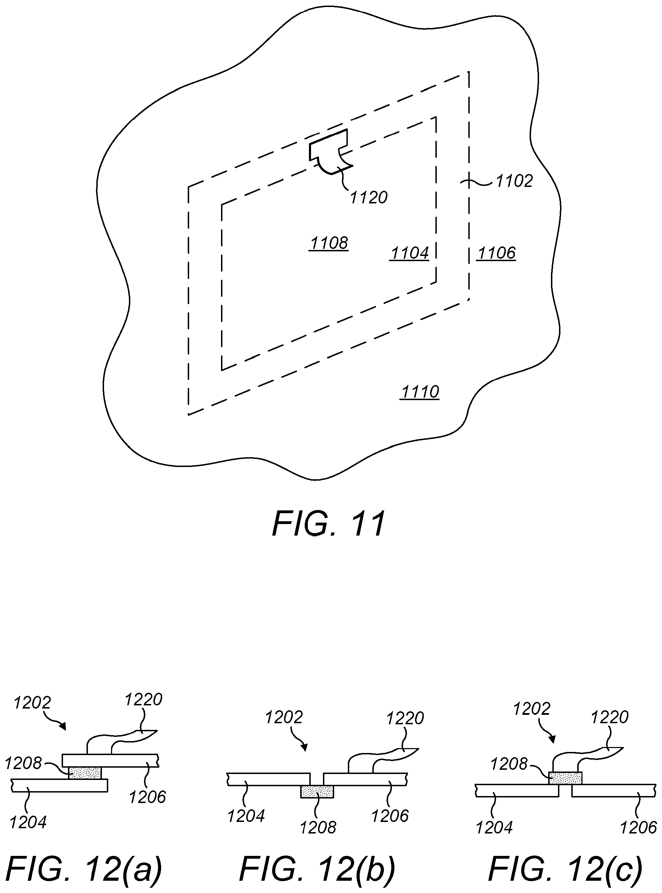

Reference is now made to figure 11. In some examples, the separation line comprises a removable tear strip 1102. The removable tear strip may be of relatively stronger material than the neighbouring portions 1104 1106. Thus, applying a force to the removable tear strip may cause the removable tear strip to preferentially be removed from the drape. The removable tear strip may be a line (whether straight, as illustrated in figure 5, or at least partly curved and/or comprising one or more corners). The removable tear strip may enclose a removable section 1108 of the drape 1110, as illustrated in figure 11.

In some examples, the separation line may comprise an adhesive join between the neighbouring portions of the drape. Examples of such adhesive joins are illustrated in figures 12a, 12b and 12c. The separation line is generally indicated at 1202. Figure 12a shows the separation line 1202 between two neighbouring portions 1204 1206 of the drape. The neighbouring portions are joined to one another by an adhesive join comprising an adhesive material 1208 disposed between overlapping portions of the neighbouring portions 1204 1206. The adhesive material 1208 may be configured to preferentially separate from one of the neighbouring portions. For example, one surface of the adhesive material 1208 may be more adhesive than another surface of the adhesive material. Such a configuration may be useful where the separation line encloses a removable section of the drape. It may be desirable for the adhesive material 1208 to preferentially remain attached to the removable section to avoid an adhesive being left around the edge of the opening thereby formed.

In another configuration, illustrated in figure 12b, the neighbouring portions 1204 1206 attach to the same side of the adhesive material 1208. The adhesive material may preferentially adhere to one of the neighbouring portions. For example, one side of the surface of the adhesive material, to which one of the neighbouring portions is adhered may be more adhesive than the other side of the surface of the adhesive material, to which the other of the neighbouring portions is adhered.

For ease of separating the neighbouring portions about the separation line, a tab may be provided for holding by a user. Suitably the accessibility portion comprises the tab. Pulling on the tab may thus cause the separation of the neighbouring portions about the separation line. One tab may be provided to one side of the separation line. Another tab may be provided to the other side of the separation line. This arrangement permits a user to pull the tabs apart so as to separate the neighbouring portions.

Since it is desirable for the outer surface of the drape to remain sterile, it is preferred if tabs are provided on those portions of the accessibility portion that are removable. Tabs may, in some examples, be provided on non-removable portions of the accessibility portion instead of or in addition to providing tabs on removable portions of the accessibility portions.

Referring again to figure 10, the fold or pleat of material may comprise the tab 1012. This arrangement enables a user to hold a portion of the accessibility portion without requiring the tab to be separately formed. Suitably, where, for example, the separation line encloses a removable portion of the drape, the fold of material comprising the tab 1012 is provided on the removable portion of the accessibility portion. In this way, a non-sterile user can hold the tab for opening the accessibility portion without affecting the sterility of the remainder of the drape.

In examples where the separation line comprises a removable tear strip, one example of which is illustrated in figure 11, a tab 1120 may be provided on the removable tear strip so as to permit a user to apply a force to the removable tear strip. In examples where the separation line comprises an adhesive join, examples of which are illustrated in figures 12a to 12c, a tab 1220 may be provided on one or more of the neighbouring portions 1204 1206 and the adhesive material 1208. Suitably, referring to the example illustrated in figure 12c, the tab 1220 is provided on the adhesive material 1208 so as to permit the adhesive material to be removed from both of the neighbouring portions 1204 1206. In this example, the tab may be adhered to the adhesive material more strongly than the adhesive material is adhered to either of the neighbouring portions. The tab may be formed unitarily with the adhesive portion.

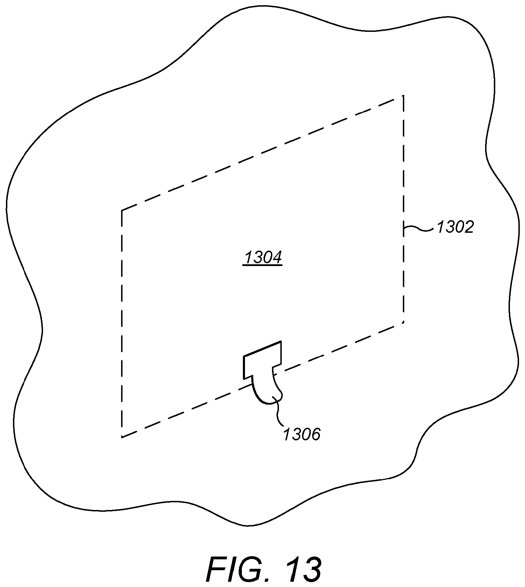

In examples where the separation line 1302 encloses a removable section 1304 of the drape, as illustrated in figure 13, a tab 1306 may be provided on the removable section 1304.

The configurations of the accessibility portions, and/or of the separation line of the accessibility portions, discussed herein may be provided in any suitable combination. Suitably the accessibility portions are formed unitarily with the drape. This approach can make the drape simpler to manufacture.

In the above discussion, access may be provided to the robot, or to at least a portion of a robot, via an accessibility portion. The accessibility portion permits an opening to be formed in the drape thereby allowing access through the drape. An alternative way of providing access to the robot, which may be used independently of the accessibility portions described herein, or together with any desired combination of the accessibility portions described herein, is to permit the drape material to be raised so as to allow access to the portion of the robot thereby uncovered.

Simply raising the drape will generally not be convenient, since the drape is likely then to need holding in place by a user for the duration of the time that access is required. There is also the difficulty of how a non-sterile person may raise the drape without causing the outer surface of the drape to become non-sterile.

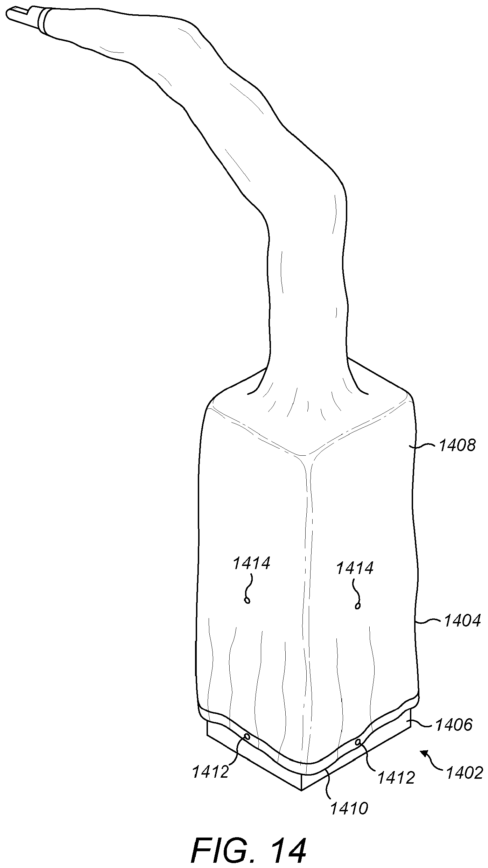

The material of the drape is flexible, to allow the drape to be unfolded or unfurled from a packaged state, and to permit the drape to be applied over the robot. The part of the drape which is generally open is the lower part of the drape (generally indicated at 1402 in figure 14). The portion of the drape terminating in the open end can be called a skirt portion 1404. Thus, referring to figure 14, it can be seen that the robot 1406 is draped by the drape 1408, wherein the drape comprises a skirt portion 1404. In a first configuration, as illustrated in figure 14, the skirt portion 1404 drapes the robot so as to provide a sterile barrier thereover.

In some examples, the drape comprises a retention arrangement configured for retaining the skirt portion in a second configuration. In the second configuration, the skirt portion exposes a portion of the surgical robot for providing access thereto. The skirt portion suitably exposes the portion of the robot by being at least partially raised relative to the robot. The skirt portion is suitably movable between the first configuration and the second configuration so as selectively to provide access to the portion of the surgical robot draped by the drape. Thus, the skirt portion can be moved into the second configuration, and then retained in that configuration by the retention arrangement. This allows a user to obtain access to the robot without needing to hold the drape out of the way for the duration that the access is required.

The drape may be configured to permit the skirt portion to be moved between the first configuration and the second configuration without thereby affecting the sterility of the drape. For example, a non-sterile member of staff may be able to move the skirt portion between the first and second configurations without affecting the sterility of the drape, such as by avoiding contact with the outer surface of the drape. This will be discussed in more detail below.

The drape will typically have little self-weight. This means that it will not fall down (i.e. return to the first configuration) when released. The drape may comprise a weight for urging the skirt portion towards the first configuration. The weight may be provided on the skirt portion or otherwise be fast with the skirt portion. Suitably, the weight is provided at or towards a lower edge of the drape, such as by being provided at or towards a lower edge of the skirt portion. This arrangement can assist in urging the drape to fully drape the robot. For example, the weight can be provided within a fold or pocket of the drape. In some examples, the weight comprises a weighted strip, the weighted strip being provided along at least a portion of the lower edge of the drape. The weighted strip may be provided in discrete lengths along the lower edge of the drape. The weighted strip may be provided all the way along the lower edge of the drape.

In some examples, a rim 1410 of the skirt portion may define the lower edge of the drape. In the second configuration, the rim of the skirt portion may be arched, or otherwise raised with respect to its position in the first configuration, so as to expose the portion of the surgical robot.

The retention arrangement may also be provided at or towards a lower edge of the drape, such as by being provided at or towards a lower edge of the skirt portion. The retention arrangement can be attached to the drape. The retention arrangement can be attached to the drape in any convenient way, such as by at least one of welding, for example hot-gas welding, vibration welding, ultrasonic welding, induction welding, infrared/laser welding and dielectric welding, and bonding, for example by an adhesive, by solvent bonding or by fusion bonding. The retention arrangement may be attached to the skirt portion 1404.

The retention arrangement may be configured to retain the skirt portion through the drape, i.e. through material of the drape. The retention arrangement may be configured to retain the skirt portion by acting through the skirt portion. For example, the retention arrangement may be configured to act magnetically. The retention arrangement may be configured to retain the skirt portion mechanically. For example via a clip and/or other mechanical retention mechanism. An example of a mechanical retention mechanism is a hook and loop fastener.

Suitably, the retention arrangement is configured for releasable engagement with a further retention arrangement for retaining the skirt portion in the second configuration. For example, where the retention arrangement is configured for retaining the skirt portion magnetically, the retention arrangement can comprise a magnetic material such as a magnet, and the further retention arrangement can comprise a magnetic material such as a magnet. Where the retention arrangement is configured for retaining the skirt portion mechanically, the retention arrangement can comprise one part of a cooperating retention mechanism, and the further retention arrangement can comprise another part of the cooperating retention mechanism, for cooperating with the first part of the mechanism. For instance, the cooperating retention mechanism can comprise a hook and loop fastener. The one part of the mechanism can comprise the hook part, and the other part of the mechanism can comprise the loop part.

In some examples, the further retention arrangement may be attached to the drape in any convenient way, such as by at least one of welding, for example hot-gas welding, vibration welding, ultrasonic welding, induction welding, infrared/laser welding and dielectric welding, and bonding, for example by an adhesive, by solvent bonding or by fusion bonding. Thus, the further retention arrangement, for example a magnetic material and/or a mechanical attachment mechanism, may be attached to a desired location of the drape during manufacturing of the drape, as the drape is being unpackaged and applied to the robot or after application of the drape to the robot. The location at which the further retention arrangement is attached to the drape can therefore be selected as desired in the particular circumstances. This provides a flexible approach to the location of the further retention arrangement. In some examples, a plurality of further retention arrangements may be provided, at least one of which is pre-applied to the drape and at least one of which is applied to the drape after manufacture of the drape, for example at the point of use of the drape.

Suitably the drape comprises a first retention feature forming at least part of the further retention arrangement.

Reference is again made to figure 14. In the illustrated example, the drape 1408 comprises a retention arrangement 1412 provided on the skirt portion 1404. The drape comprises a further retention arrangement 1414. The further retention arrangement is provided for cooperating with the retention arrangement so as to retain the skirt portion in the second configuration. As shown in figure 14, the further retention arrangement can be provided on a portion of the drape above the skirt portion 1404.

In the illustrated example, the further retention arrangement comprises a first retention feature 1414. The first retention feature may be attached to the drape in any convenient way, such as by at least one of welding, for example hot-gas welding, vibration welding, ultrasonic welding, induction welding, infrared/laser welding and dielectric welding, and bonding, for example by an adhesive, by solvent bonding or by fusion bonding. The first retention feature may be held fast relative to material of the drape in a fold or pocket of the drape.

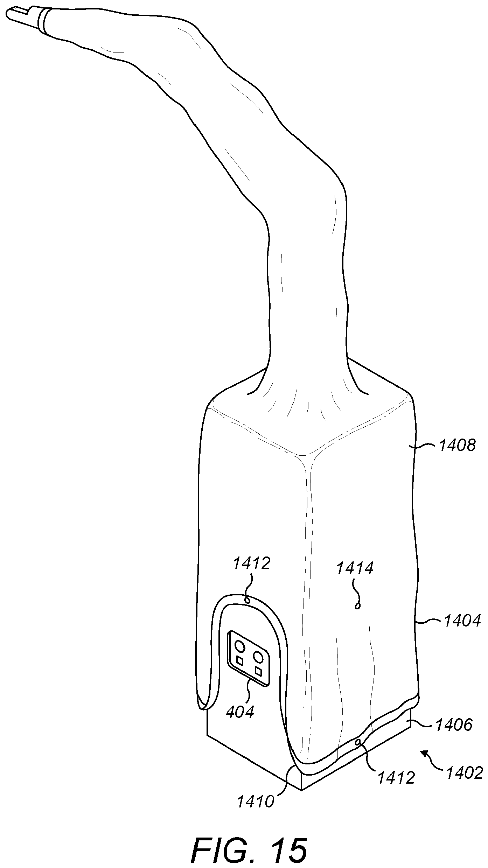

For example, the retention arrangement 1412 may comprise a first magnetic material. The retention arrangement 1412 may comprise a magnet. The further retention arrangement 1414 may comprise a magnetic material. The further retention arrangement 1414 may comprise a magnet. In some examples, the further retention arrangement comprises a first retention feature which comprises a magnetic material such as a magnet. This configuration permits the retention arrangement to couple magnetically to the further retention arrangement, for example by coupling to the first retention feature. With reference now to figure 15, it can be seen that moving the retention arrangement 1412 so as to couple to the further retention arrangement 1414 causes the skirt portion to move to the second configuration. In this second configuration of the skirt portion, access is provided to a portion of the robot which is draped by the drape when the skirt portion is in the first configuration. For example, figure 15 illustrates that, when the skirt portion is in the second configuration, access is provided to the connector panel 404. In the first configuration of the skirt portion (illustrated in figure 14) the connector panel 404 is covered by the drape.

The drape need not comprise the further retention arrangement 1414. In some examples, the further retention arrangement may be provided on, or as part of, the robot to be draped by the drape. For example, where the further retention arrangement comprises a magnetic material, this magnetic material may be provided at a suitable location on the robot, for example on the cart. The robot may comprise the at least a portion of the further retention arrangement. The magnetic material of the retention arrangement may couple to the magnetic material of the further retention arrangement through the drape. This permits the skirt portion to be retained in the second configuration without needing additional attachment features to be provided on the drape. It also has the benefit of enabling the further retention feature to be located on the robot at a location which provides suitable access to the robot when the skirt portion is in the second configuration. For example, the height on the robot at which the further retention arrangement is provided can be selected to ensure that when the skirt portion is in the second configuration, the rim of the skirt portion is raised sufficiently to permit access to a particular location on the robot, such as the connector panel 404.

In some examples, the further retention arrangement may comprise a second retention feature. The second retention feature may be provided on (or as part of) the robot to be draped by the drape. Thus, the further retention arrangement may comprise a first retention feature on the drape, such as a magnetic material, and a second retention feature on the robot to be draped by the drape, such as a magnetic material. This configuration permits a selection of the first and second retention feature to which the retention arrangement may be coupled so as to retain the skirt portion in the second configuration. This approach has the benefit of flexibility of selection of the most appropriate retention feature to which to couple the retention arrangement.

The first retention feature and the second retention feature need not couple to the retention arrangement in the same manner. For example, the retention arrangement may be configured to be mechanically and magnetically attachable to the further retention arrangement. The retention arrangement may comprise a magnetic material and a mechanical attachment arrangement. Thus, where the first retention feature on the drape comprises a cooperating mechanical attachment arrangement, and the second retention feature on the robot comprises a magnetic material, the retention arrangement may be coupled to either the first retention feature or the second retention feature. The user may select the respective retention feature to which the retention arrangement is to be coupled.

In some examples, the retention arrangement comprises one part of a hook and look fastener, and the second retention feature comprises the cooperating part of the hook and look fastener.

A further retention arrangement need not be provided in all examples. For example, the retention arrangement may comprise a mechanical attachment mechanism such as one or more of a clip, hook and clamp, configured to be releasably engageable with a portion of the drape. For example, where the retention arrangement comprises a clip, the clip may be attached to a portion of the drape by clipping the clip to the material of the drape.

As discussed above, it is desirable for the drape to be configured such that a non-sterile member of staff may move the skirt portion between the first and second configurations without adversely affecting the sterility of the drape, for example the outer, sterile, surface of the drape.

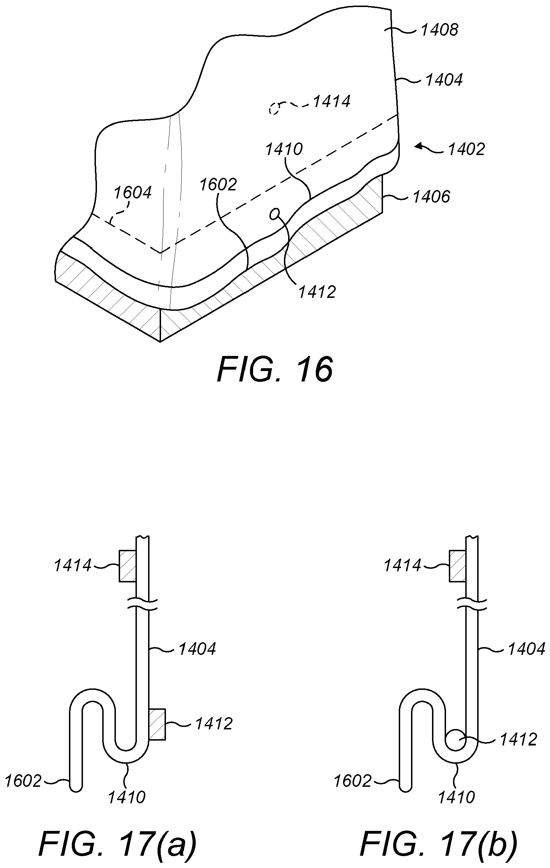

In some examples, the drape comprises a fold or tab, whereby a user can hold the drape via the fold or tab without thereby affecting the sterility of the remainder of the drape. Suitably, the fold or tab is provided at or towards the lower edge of the drape. The fold or tab may be provided so as to extend below an edge of the drape, such as the rim of the skirt portion. This is illustrated in figure 16. In the illustrated example, the drape comprises a fold of material behind the lower portion of the skirt portion 1404. The fold or pleat 1602 extends downwardly past the end of the rim 1410 of the skirt portion. An upper edge of the fold (which is behind the outer surface of the drape, as viewed in figure 16) is indicated in dotted lines 1604. This configuration enables a non-sterile person to cause the skirt portion 1404 of the drape to move between the first and second configurations by holding the fold 1602. The non-sterile person need not touch the remainder of the drape. In particular, the non-sterile person need not touch the sterile outer surface of the drape. Thus, the outer surface of the drape can remain sterile, even where the drape is moved by a non-sterile person. This advantageously permits non-sterile staff to obtain access to the robot when it is draped by the drape. The further retention element 1414 is shown in dashed lines in figure 16 to indicate that it may be on the front surface of the drape, within a fold or pocket in the drape, or on the rear surface of the drape.

The retention arrangement may comprise the fold or tab.

The retention arrangement may comprise the weight. The retention arrangement may comprise the weighted strip. In some examples, the weight may be a magnetic material. The weight may be a magnet. This can simplify the construction of the drape, since there is no need for the magnetic material and the weight to be provided separately. This can simplify the manufacturing process, and can reduce the amount of materials used in the construction of the drape.

Some examples of drapes comprising retention arrangements will now be described with reference to figures 17 to 20. In these examples, the retention arrangement comprises a magnet, and the further retention arrangement comprises a magnet. The magnet of the further retention arrangement is shown as being attached to the rear surface (i.e. the non-sterile side) of the drape. In other examples the magnet may be attached to the front surface (i.e. the sterile side) of the drape, or it may be held fast with respect to the drape in a fold or pocket of the drape. It will be understood that the configuration which is adopted is a matter of choice in the construction of the drape, and does not affect the underlying operation of the retention of the skirt portion. The provision of the further retention arrangement on the rear surface of the drape, or in a fold or pocket of the drape, can be advantageous since in such a configuration the further retention arrangement is less likely to catch on anything adjacent the draped robot in the OR. The provision of the further retention arrangement in a fold or pocket of the drape may decrease the likelihood of the further retention arrangement catching on the robot. Similarly, the magnet of the retention arrangement may be provided on a front surface, rear surface or in a fold or pocket of the drape. Providing the magnet on the rear surface or in a fold or pocket of the drape can reduce the likelihood that the magnet catches on anything adjacent the draped robot in the OR. The provision of the retention arrangement in a fold or pocket of the drape may decrease the likelihood of the retention arrangement catching on the robot.

In these examples, the weight is not separately shown. The magnet and the weight may be one and the same element. Alternatively, or additionally, one or more weights may be provided adjacent and/or spaced from the magnet.

Figures 17a and 17b show cross-sectional views of examples of a drape comprising a fold or pleat of material. In figures 17a and 17b, the non-sterile side of the drape is to the left in the drawings. In figure 17a, a further retention arrangement 1414 is attached to an upper portion of the drape. A retention arrangement 1412 is attached to the front surface of the skirt portion 1404. The retention arrangement 1412 is attached to the skirt portion 1404 adjacent the rim 1410. A fold or pleat of material of the drape forms a depending fold 1602, which extends lower than the rim 1410. Thus a non-sterile person may hold the fold 1602 and move the retention arrangement 1412 for coupling with the further retention arrangement 1414. When so coupled, the fold will hang below the level of the raised rim 1410 and so will not contact the sterile portion of the drape. Thus the sterility of the drape can be maintained. The strength of the engagement between the retention arrangement and the further retention arrangement holds the skirt portion in the second configuration against the action of the weight, which urges the skirt portion towards the first configuration.

The skirt portion can be moved from the second, raised, configuration to the first, lowered, configuration. The user can hold the fold 1602 to cause the release of the coupling between the retention arrangement 1412 and the further retention arrangement 1414. For example, the magnets can be pulled apart. On release of the fold, the skirt portion will move towards the first configuration under action of the weight.

Figure 17b illustrates an example in which the magnet is held fast relative to the skirt portion 1404 in a fold of the drape. The operation of this example is similar to that of figure 17a, as will be understood.

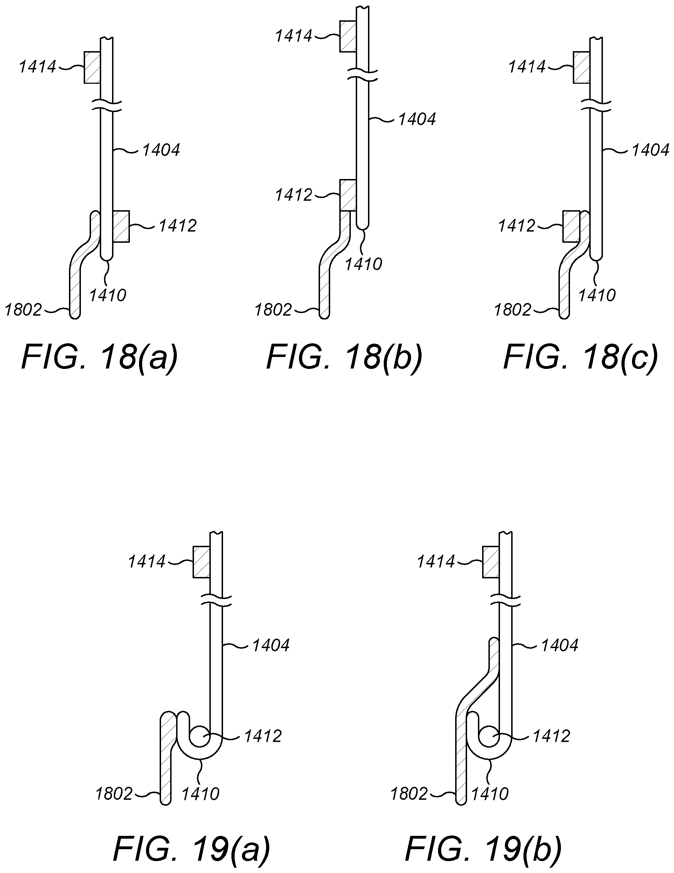

Figures 18a to 18c illustrate examples where a tab is provided on the drape. In figure 18a, the retention arrangement 1412 comprises a magnet attached to the front surface of the skirt portion 1404. A tab 1802 is attached to a rear surface of the skirt portion 1404. The tab 1802 extends below the level of the rim 1410 of the skirt portion. The configuration of the drape in figure 18b is similar to that in figure 18a, except that in this configuration, the retention arrangement is attached to the rear of the skirt portion 1404. The tab 1802 may attach to the retention arrangement, as illustrated, or it may attach to a neighbouring portion of the rear of the skirt portion, for example to a region adjacent the retention arrangement. As illustrated in figure 18c, the retention arrangement 1412 may attach to the tab 1802. In some examples, the tab and the retention arrangement are formed unitarily. This can simplify the manufacturing process. The operation of the drape is similar to that of the drape examples illustrated in figure 17, as will be understood.

Figures 19a and 19b illustrate examples in which a fold and a tab may be provided. In figure 19a, the retention arrangement 1412 is held fast relative to the drape in a fold of the drape. The bottom of the fold defines the rim 1410 of the skirt portion 1404. The tab 1802 is provided attached to the rear of the fold. The example shown in figure 19b is similar to that of figure 19a, except that in this example the tab 1802 is provided attached to a portion of the rear of the drape that is above the fold. In both examples, the tab 1802 extends below the level of the rim 1410.

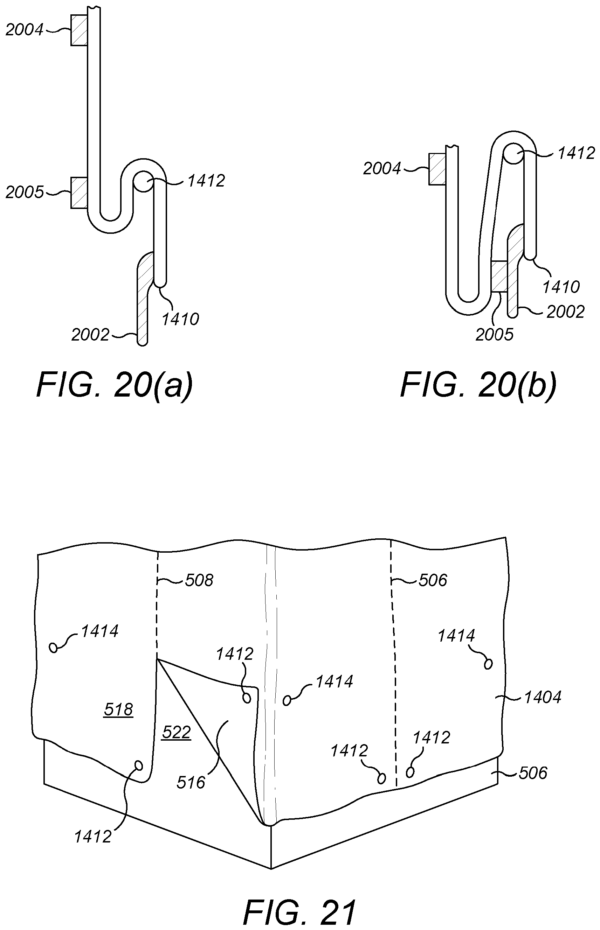

Figures 20a and 20b show another example of a drape comprising a retention arrangement. In this example, a plurality of further retention arrangements 2004 2005 are provided for releasable coupling to the retention arrangement 1412. A tab 2002 is provided attached to the skirt portion and which extends below the lower edge of the skirt portion. In some examples, the retention arrangement 1412 is configured to couple to one of the further retention arrangements 2005, to retain the skirt portion in the first configuration, as illustrated in figure 20a. The retention arrangement 1412 can be moved so as to couple to the other of the further retention arrangements 2004, to retain the skirt portion in the second configuration, as illustrated in figure 20b.

As discussed above, the retention arrangement need not couple to a further retention arrangement when the skirt portion is in the first configuration. The plurality of further retention arrangements may be so located as to retain the skirt portion in different configurations, each of which enable access to be provided to the robot to be draped by the drape.

Additional further retention arrangements may be provided as desired, for example at different heights and/or locations on the drape. This can enable the skirt portion to be retained in a number of different configurations, which can, for example, provide access to different portions of the robot. For example, one such raised configuration may retain the skirt portion such that the rim of the skirt portion is higher than in another such raised configuration. This can therefore provide access to a portion of the robot that may be higher up the robot.

In some examples, the drape may comprise more than one retention arrangement. Each of the plurality of retention arrangements may be provided in the drape at a location spaced from the other retention arrangement(s). The retention arrangements may be spaced from one another in a direction about which the drape can be selectively oriented to drape the surgical robot. In this way, at each orientation at which the drape can drape the surgical robot, one of the retention arrangements can retain the skirt portion so as to provide access to a desired portion of the surgical robot. In some examples, the drape comprises a number of retention arrangements corresponding to the number of orientations at which the robot can be draped by the drape. The retention arrangements may be equally spaced about the drape. Thus, at whichever orientation the drape is applied, one of the retention arrangements may be located so as to provide access to the desired portion of the robot (such as a face of the cart).

In the example of a cart with four faces, as illustrated in figures 4a and 4b, the drape may be provided with four such retention arrangements. Since the cart as illustrated is generally square in horizontal cross-section, the drape may be provided with the four such retention arrangements being equally spaced about the drape.

The drape may comprise a number of further retention arrangements corresponding to the number of retention arrangements. For example, where the drape comprises four retention arrangements, the drape may comprise four further retention arrangements. Where additional further retention arrangements are provided, these may be provided in respect of each of the retention arrangements, or a subset thereof.

In some examples, a drape may comprise at least one accessibility portion and at least one retention arrangement. The accessibility portion and the retention arrangement may be of any type described herein, in any combination. In some examples a drape may comprise the same number of accessibility portions and retention arrangements.

In some examples, at least one of the neighbouring portions may comprise the retention arrangement. In this way, the neighbouring portion may be retained in a configuration that permits access to the robot. For example, the neighbouring portion may be retained to one side, thereby permitting easier access through the opening in the drape formed by the separation of the neighbouring portions about the separation line. In another example, the neighbouring portion may be retained in a raised configuration. In at least some examples, one or both neighbouring portion in each accessibility portion may comprise a respective weight.

An example of a drape comprising a retention arrangement and an accessibility portion is illustrated in figure 21. The drape comprises a plurality of accessibility portions, each of which comprises a separation line (two of which are shown at 506 and 508). As illustrated two neighbouring portions 516 518 are separated about one of the separation lines 508, thereby forming an opening 522 through which access to the robot may be provided. The right hand neighbouring portion 516 comprises a retention arrangement 1412 towards a lower edge thereof. (Here 'lower' refers to the position in a configuration in which the neighbouring portion drapes the robot, before separation of the accessibility portion about the separation line.) The retention arrangement may be configured to couple to a further retention arrangement provided on the drape and/or on the robot draped by the drape.

In some examples the drape may comprise a retention arrangement and a cooperating further retention arrangement corresponding to each accessibility portion. In the illustrated example, the drape comprises multiple pairs of retention arrangements and further retention arrangements. As illustrated, there is a retention arrangement and a corresponding further retention arrangement provided for each neighbouring portion of each accessibility portion. This configuration permits either or both of the neighbouring portions to be retained in a configuration in which the neighbouring portion exposes a portion of the surgical robot for providing access thereto. Suitably the skirt portion comprises the neighbouring portions.

The example illustrated in figure 21 may comprise one or more of a fold and a tab, as described above.

The drape described herein could be used for non-surgical robotic purposes. For example, it could be used in robotic systems, or systems more generally, in which it is desirable to provide a barrier which accommodates relative motion of parts of the barrier. Such a barrier might be a barrier to fluid flow and/or a barrier to particulate matter, for example particulate matter entrained in a flow of fluid such as air. Such a barrier can therefore be used to provide effective protection from chemicals, material filings and/or dust.

In view of the foregoing description it will be evident to a person skilled in the art that various modifications may be made within the scope of the invention. The scope of the invention is defined by the appended claims.

## Claims

1. A surgical robot drape (114) for providing a sterile barrier over a portion of a surgical robot (100), the drape (114) comprising:
a plurality of accessibility portions spaced apart from one another in the drape (114) for providing selective access through the drape (114) at the respective locations of the accessibility portions, each accessibility portion comprising a separation line (506, 508) joining neighbouring portions (512, 514, 516, 518) of the drape and being configured to permit the separation of the neighbouring portions (512, 514, 516, 518) of the drape (114) about the separation line (506, 508) for allowing access through an opening (522) thereby formed in the drape.

2. A surgical robot drape (114) according to claim 1, in which the drape (114) comprises the same number of accessibility portions as a number of orientations at which a surgical robot (100) can be draped by the drape (114), and/or in which the accessibility portions are equally spaced about the drape (114), in a direction about which the drape (114) can be selectively oriented to drape the surgical robot (100).

3. A surgical robot drape (114) according to any preceding claim, in which each accessibility portion is configured to preferentially tear along the separation line (506, 508) under action of a force.

4. A surgical robot drape (114) according to any preceding claim, in which the separation line (506, 508) comprises one or more of:
a weakened portion of material relative to the neighbouring portions (512, 514, 516, 518) of the drape (114);
material thinner than that of the neighbouring portions (512, 514, 516, 518);
perforations (905), in which the perforations (905) optionally extend through the material;
a removable tear strip (1102) of relatively stronger material than the neighbouring portions (1104, 1106); and
an adhesive join between the neighbouring portions (1204, 1206).

5. A surgical robot drape (114) according to any preceding claim, in which the separation line (506, 508) at least one of:
encloses a removable section (707) of the drape (114);
extends to an edge (510) of the drape (114).

6. A surgical robot drape (114) according to any preceding claim, in which the accessibility portion:
is formed unitarily with the drape (114), and/or
comprises one or more of:
a portion of material overlapping (1010) with the separation line (1004), in which the overlapping material (1010) is optionally formed by a fold in the material; and
a tab (1012) for holding by a user.

7. A surgical robot drape (114) according to any of claims 4 to 6, in which the adhesive join comprises a portion of separation material (1208) adhesively joined to each of the neighbouring portions (1204, 1206).

8. A surgical robot drape (114) according to any of claims 6 to 7, in which the fold in the material comprises the tab (1012).

9. A surgical robot drape (114) according to any preceding claim, in which the drape (114) comprises:
a flexible sheet having a skirt portion (1404) configured to, in a first configuration, drape the portion of the surgical robot (100) to provide a sterile barrier thereover; and
a retention arrangement (1412) configured for retaining the skirt portion (1404) in a second configuration in which the skirt portion (1404) exposes the portion of the surgical robot (100) for providing access thereto.

10. A surgical robot drape (114) according to claim 9, comprising one or more of:
a weight for urging the skirt portion (1404) towards the first configuration;
a fold (1602) or tab (1802), whereby a user can hold the drape (114) via the fold (1602) or tab (1802) without thereby affecting the sterility of the remainder of the drape (114); and
a plurality of retention arrangements (1412, 1414).

11. A surgical robot drape (114) according to claim 9 or claim 10, in which the retention arrangement (1412) is one or more of:
provided at or towards a lower edge of the drape (114);
configured for retaining the skirt portion (1404) at least one of mechanically and magnetically; and
configured for releasable engagement with a further retention arrangement (1414) for retaining the skirt portion (1404) in the second configuration.

12. A surgical robot drape (114) according to claim 10 or claim 11, in which the retention arrangement (1412) comprises the weight and/or the fold (1602) or tab (1802).

13. A surgical robot drape (114) according to any of claims 10 to 12, in which the drape (114) comprises the same number of retention arrangements (1412, 1414) as a number of orientations at which a surgical robot (100) can be draped by the drape (114) and/or in which the retention arrangements (1412, 1414) are equally spaced about the drape (114), in a direction about which the drape (114) can be selectively oriented to drape (114) the surgical robot (100).

14. A surgical robot drape (114) according to any of claims 9 to 13, in which at least one of the neighbouring portions of each accessibility portion comprises one or more of:
a respective retention arrangement (1412); and
a respective weight.

15. A surgical robotic system comprising a surgical robot (100) and a surgical robot drape (114) according to any preceding claim, in which the drape (114) is disposed about the surgical robot (100) for providing a sterile barrier over at least a portion thereof.

## Patentansprüche

1. Abdecktuch (114) für einen chirurgischen Roboter zum Bereitstellen einer sterilen Barriere über einem Abschnitt eines chirurgischen Roboters (100), das Abdecktuch (114) umfassend:
eine Vielzahl von Zugangsabschnitten, die in dem Abdecktuch (114) voneinander beabstandet sind, um selektiven Zugang durch das Abdecktuch (114) an den jeweiligen Orten der Zugangsabschnitte bereitzustellen, wobei jeder Zugangsabschnitt eine Trennlinie (506, 508) umfasst, die benachbarte Abschnitte (512, 514, 516, 518) des Abdecktuchs verbindet und so konfiguriert ist, dass sie die Trennung der benachbarten Abschnitte (512, 514, 516, 518) des Abdecktuchs (114) um die Trennlinie (506, 508) ermöglicht, um Zugang durch eine Öffnung (522) zu ermöglichen, die dadurch in dem Abdecktuch gebildet wird.

2. Abdecktuch (114) für einen chirurgischen Roboter nach Anspruch 1, wobei das Abdecktuch (114) die gleiche Anzahl von Zugangsabschnitten wie eine Anzahl von Ausrichtungen umfasst, an denen ein chirurgischer Roboter (100) durch das Abdecktuch (114) abdeckt werden kann, und/oder wobei die Zugangsabschnitte über das Abdecktuch (114) gleichmäßig voneinander beabstandet sind, in einer Richtung, in der das Abdecktuch (114) selektiv ausgerichtet werden kann, um den chirurgischen Roboter (100) abzudecken.

3. Abdecktuch (114) für einen chirurgischen Roboter nach einem der vorhergehenden Ansprüche, wobei jeder Zugangsabschnitt so konfiguriert ist, dass er vorzugsweise entlang der Trennlinie (506, 508) unter Wirkung einer Kraft reißt.

4. Abdecktuch (114) für einen chirurgischen Roboter nach einem der vorhergehenden Ansprüche, wobei die Trennlinie (506, 508) eines oder mehrere der Folgenden umfasst:
einen geschwächten Materialabschnitt in Bezug auf die benachbarten Abschnitte (512, 514, 516, 518) des Abdecktuchs (114);
Material, das dünner ist als das der benachbarten Abschnitte (512, 514, 516, 518);
Perforationen (905), wobei sich die Perforationen (905) optional durch das Material erstrecken;
einen entfernbaren Aufreißstreifen (1102) aus relativ stärkerem Material als die benachbarten Abschnitte (1104, 1106); und
eine Klebeverbindung zwischen den benachbarten Abschnitten (1204, 1206).

5. Abdecktuch (114) für einen chirurgischen Roboter nach einem der vorhergehenden Ansprüche, wobei die Trennlinie (506, 508) zumindest:
einen entfernbaren Abschnitt (707) des Abdecktuchs (114) umschließt; und/oder
sich zu einer Kante (510) des Abdecktuchs (114) hin erstreckt.

6. Abdecktuch (114) für einen chirurgischen Roboter nach einem der vorhergehenden Ansprüche, wobei der Zugangsabschnitt:
einstückig mit dem Abdecktuch (114) ausgebildet ist, und/oder
eines oder mehrere der Folgenden umfasst:
einen Materialabschnitt, der mit der Trennlinie (1004) überlappt (1010), wobei das überlappende Material (1010) optional durch eine Falte in dem Material ausgebildet wird; und
eine Lasche (1012) zum Halten durch einen Benutzer.

7. Abdecktuch (114) für einen chirurgischen Roboter nach einem der Ansprüche 4 bis 6, wobei die Klebeverbindung einen Abschnitt aus Trennmaterial (1208) umfasst, das mit jedem der benachbarten Abschnitte (1204, 1206) haftend verbunden ist.

8. Abdecktuch (114) für einen chirurgischen Roboter nach einem der Ansprüche 6 bis 7, wobei die Falte in dem Material die Lasche (1012) umfasst.

9. Abdecktuch (114) für einen chirurgischen Roboter nach einem der vorhergehenden Ansprüche, wobei das Abdecktuch (114) Folgendes umfasst:
eine flexible Folie mit einem Randabschnitt (1404), der so konfiguriert ist, dass er in einer ersten Konfiguration den Abschnitt des chirurgischen Roboters (100) abdeckt, um eine sterile Barriere über diesem bereitzustellen; und
eine Halteanordnung (1412), die zum Halten des Randabschnitts (1404) in einer zweiten Konfiguration konfiguriert ist, in der der Randabschnitt (1404) den Abschnitt des chirurgischen Roboters (100) freilegt, um Zugang zu diesem bereitzustellen.

10. Abdecktuch (114) für einen chirurgischen Roboter nach Anspruch 9, umfassend eines oder mehrere von:
einem Gewicht zum Drängen des Randabschnitts (1404) in Richtung der ersten Konfiguration;
einer Falte (1602) oder Lasche (1802), wobei ein Benutzer das Abdecktuch (114) mittels der Falte (1602) oder Lasche (1802) halten kann, ohne dadurch die Sterilität des Rests des Abdecktuchs (114) zu beeinträchtigen; und
einer Vielzahl von Halteanordnungen (1412, 1414).

11. Abdecktuch (114) für einen chirurgischen Roboter nach Anspruch 9 oder Anspruch 10, wobei die Halteanordnung (1412):
an oder in Richtung einer unteren Kante des Abdecktuchs (114) bereitgestellt ist; und/oder
zum zumindest einen von dem mechanischen und magnetischen Halten des Randabschnitts (1404) konfiguriert ist; und/oder
für einen lösbaren Eingriff mit einer weiteren Halteanordnung (1414) zum Halten des Randabschnitts (1404) in der zweiten Konfiguration konfiguriert ist.

12. Abdecktuch (114) für einen chirurgischen Roboter nach Anspruch 10 oder Anspruch 11, wobei die Halteanordnung (1412) das Gewicht und/oder die Falte (1602) oder Lasche (1802) umfasst.

13. Abdecktuch (114) für einen chirurgischen Roboter nach einem der Ansprüche 10 bis 12, wobei das Abdecktuch (114) die gleiche Anzahl von Halteanordnungen (1412, 1414) aufweist wie eine Anzahl von Ausrichtungen, an denen ein chirurgischer Roboter (100) durch das Abdecktuch (114) abdeckt werden kann, und/oder wobei die Halteanordnungen (1412, 1414) über das Abdecktuch (114) gleichmäßig voneinander beabstandet sind, in einer Richtung, in der das Abdecktuch (114) selektiv ausgerichtet werden kann, um den chirurgischen Roboter (100) abzudecken (114).

14. Abdecktuch (114) für einen chirurgischen Roboter nach einem der Ansprüche 9 bis 13, wobei zumindest einer der benachbarten Abschnitte jedes Zugangsabschnitts eines oder mehrere der Folgenden umfasst:
eine jeweilige Halteanordnung (1412); und
ein jeweiliges Gewicht.

15. Chirurgisches Robotersystem, umfassend einen chirurgischen Roboter (100) und ein Abdecktuch (114) für einen chirurgischen Roboter nach einem der vorhergehenden Ansprüche, wobei das Abdecktuch (114) um den chirurgischen Roboter (100) angeordnet ist, um eine sterile Barriere über zumindest einen Abschnitt von diesem bereitzustellen.

## Revendications

1. Drapage de robot chirurgical (114) pour fournir une barrière stérile sur une portion d'un robot chirurgical (100), le drapage (114) comprenant :
une pluralité de portions d'accessibilité espacées les unes des autres dans le drapage (114) pour fournir un accès sélectif à travers le drapage (114) au niveau des emplacements respectifs des portions d'accessibilité, chaque portion d'accessibilité comprenant une ligne de séparation (506, 508) reliant des portions voisines (512, 514, 516, 518) du drapage et étant configurée pour permettre la séparation des portions voisines (512, 514, 516, 518) du drapage (114) autour de la ligne de séparation (506, 508) pour permettre l'accès à travers une ouverture (522) ainsi formée dans le drapage.

2. Drapage de robot chirurgical (114) selon la revendication 1, dans lequel le drapage (114) comprend le même nombre de portions d'accessibilité qu'un nombre d'orientations selon lesquelles un robot chirurgical (100) peut être drapé par le drapage (114), et/ou dans lequel les portions d'accessibilité sont espacées de manière égale autour du drapage (114), dans une direction autour de laquelle le drapage (114) peut être sélectivement orienté pour draper le robot chirurgical (100).

3. Drapage de robot chirurgical (114) selon l'une quelconque des revendications précédentes, dans lequel chaque portion d'accessibilité est configurée pour se déchirer de préférence le long de la ligne de séparation (506, 508) sous l'action d'une force.

4. Drapage de robot chirurgical (114) selon l'une quelconque des revendications précédentes, dans lequel la ligne de séparation (506, 508) comprend un ou plusieurs parmi :
une portion affaiblie de matériau par rapport aux portions voisines (512, 514, 516, 518) du drapage (114) ;
un matériau plus mince que celui des portions voisines (512, 514, 516, 518) ;
des perforations (905), dans lequel les perforations (905) s'étendent facultativement à travers le matériau ;
une bande déchirable amovible (1102) de matériau relativement plus résistant que les portions voisines (1104, 1106) ; et
une couture adhésive entre les portions voisines (1204, 1206) .

5. Drapage de robot chirurgical (114) selon l'une quelconque des revendications précédentes, dans lequel la ligne de séparation (506, 508) réalise au moins l'une des actions parmi :
enfermer une section amovible (707) du drapage (114) ;
s'étendre jusqu'à un bord (510) du drapage (114).

6. Drapage de robot chirurgical (114) selon l'une quelconque des revendications précédentes, dans lequel la portion d'accessibilité :
est formée de manière unitaire avec le drapage (114), et/ou
comprend un ou plusieurs parmi :
une portion de matériau chevauchant (1010) la ligne de séparation (1004), dans lequel le matériau chevauchant (1010) est facultativement formé par un pli dans le matériau ; et
une languette (1012) destinée à être maintenue par un utilisateur.

7. Drapage de robot chirurgical (114) selon l'une quelconque des revendications 4 à 6, dans lequel la couture adhésive comprend une portion de matériau de séparation (1208) reliée de manière adhésive à chacune des portions voisines (1204, 1206).

8. Drapage de robot chirurgical (114) selon l'une quelconque des revendications 6 et 7, dans lequel le pli dans le matériau comprend la languette (1012).

9. Drapage de robot chirurgical (114) selon l'une quelconque des revendications précédentes, dans lequel le drapage (114) comprend :
une feuille souple ayant une portion de jupe (1404) configurée pour, dans une première configuration, draper la portion du robot chirurgical (100) pour fournir une barrière stérile sur celle-ci ; et
un agencement de retenue (1412) configuré pour retenir la portion de jupe (1404) dans une deuxième configuration dans laquelle la portion de jupe (1404) expose la portion du robot chirurgical (100) pour fournir un accès à celle-ci.

10. Drapage de robot chirurgical (114) selon la revendication 9, comprenant un ou plusieurs parmi :
un poids pour pousser la portion de jupe (1404) vers la première configuration ;
un pli (1602) ou une languette (1802), moyennant quoi un utilisateur peut maintenir le drapage (114) par l'intermédiaire du pli (1602) ou de la languette (1802) sans affecter ainsi la stérilité du reste du drapage (114) ; et
une pluralité d'agencements de retenue (1412, 1414).

11. Drapage de robot chirurgical (114) selon la revendication 9 ou la revendication 10, dans lequel l'agencement de retenue (1412) est un ou plusieurs parmi :
fourni au niveau de ou vers un bord inférieur du drapage (114) ;
configuré pour retenir la portion de jupe (1404) au moins d'une manière parmi mécanique et magnétique ; et
configuré pour venir en prise de manière libérable avec un autre agencement de retenue (1414) afin de retenir la portion de jupe (1404) dans la deuxième configuration.

12. Drapage de robot chirurgical (114) selon la revendication 10 ou la revendication 11, dans lequel l'agencement de retenue (1412) comprend le poids et/ou le pli (1602) ou la languette (1802).

13. Drapage de robot chirurgical (114) selon l'une quelconque des revendications 10 à 12, dans lequel le drapage (114) comprend le même nombre d'agencements de retenue (1412, 1414) qu'un nombre d'orientations selon lesquelles un robot chirurgical (100) peut être drapé par le drapage (114) et/ou dans lequel les agencements de retenue (1412, 1414) sont espacés de manière égale autour du drapage (114), dans une direction autour de laquelle le drapage (114) peut être orienté sélectivement pour draper (114) le robot chirurgical (100).

14. Drapage de robot chirurgical (114) selon l'une quelconque des revendications 9 à 13, dans lequel l'au moins une des portions voisines de chaque portion d'accessibilité comprend un ou plusieurs parmi :
un agencement de retenue (1412) respectif ; et
un poids respectif.

15. Système robotique chirurgical comprenant un robot chirurgical (100) et un drapage de robot chirurgical (114) selon l'une quelconque des revendications précédentes, dans lequel le drapage (114) est disposé autour du robot chirurgical (100) pour fournir une barrière stérile sur au moins une portion de celui-ci.
